(19) Europäisches Patentamt
European Patent Office
Office européen des brevets

(11) Publication number : **0 515 622 B1**

(12) **EUROPEAN PATENT SPECIFICATION**

(45) Date of publication of patent specification : **20.07.94 Bulletin 94/29**

(21) Application number : **92900866.2**

(22) Date of filing : **09.12.91**

(86) International application number : **PCT/EP91/02351**

(87) International publication number : **WO 92/10107 25.06.92 Gazette 92/14**

(51) Int. Cl.$^5$ : **A23L 1/226,** C11B 9/00, A24B 15/40, A23G 3/30, C07D 317/10, C07D 317/00, A24B 15/30

# (54) ORGANOLEPTIC COMPOSITIONS.

(30) Priority : **12.12.90 US 626348**
**12.12.90 US 626354**

(43) Date of publication of application : **02.12.92 Bulletin 92/49**

(45) Publication of the grant of the patent : **20.07.94 Bulletin 94/29**

(84) Designated Contracting States : **CH DE ES FR GB IT LI NL**

(56) References cited :
**EP-A- 0 033 928**
**EP-A- 0 150 398**
**US-A- 3 818 107**
**US-A- 3 879 425**
**US-A- 4 532 944**
**US-A- 4 538 628**

(73) Proprietor : **GIVAUDAN-ROURE (INTERNATIONAL) S.A.**
**5, chemin de la Parfumerie**
**CH-1214 Vernier, Genève (CH)**

(72) Inventor : **ANDERSON, Denise, A.**
**68 Montague Street, Apt. 5C**
**Brooklyn, NY 11201 (US)**
Inventor : **CHRISTENSON, Philip, A.**
**118 Busteed Drive**
**Midland Park, NJ 07432 (US)**
Inventor : **RIKER, Paul, J.**
**44 South Main Street, Apt. 5J**
**Lodi, NJ 07644 (US)**
Inventor : **YURECKO, John, M., Jr.**
**J4 Quincy Circle**
**Dayton, NJ 08810 (US)**

(74) Representative : **Cottong, Norbert A. et al**
**Grenzacherstrasse 124**
**Postfach 3255**
**CH-4002 Basel (CH)**

EP 0 515 622 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to organoleptic compositions. More particularly, it relates to flavor and fragrance compositions which contain one or more dicarboalkoxy dioxolane derivatives which are normally odorless but which upon thermolysis, hydrolysis or both, release an odorant or flavorant molecule.

Flavor additives have long been used to flavor a wide variety of consumer products, particularly tobacco products, foodstuffs, and gums. Flavor additives in such products may be used to mask or attenuate undesirable flavors or odorants, and to enhance existing flavors or odors, or to provide additional flavors or odors not initially present in the consumer product.

A principal strategy currently employed to impart flavors or odors to consumer products is the admixing of the flavorant chemicals within a matrix that slows or prevents their release until the product is pyrolyzed, heated, masticated or wetted. Alternatively, the flavoring chemical may be covalently bound to an auxilliary component to form a higher molecular weight molecule of low volatilitiy. The flavorant is then released upon pyrolysis, heating or solvolysis of the tobacco or food product. For example, European Patent Publication 186, 502 described the use of a plastic capsule that releases flavorants when mechanically crushed.

U.S. Patent No. 4,001,438 described flavor compositions for use in orally utilizable compositions which may be either chewing gum compositions, chewable medicinal tablets, chewing tobacco or toothpaste. The flavor is controllably released from the flavor compositions over an extended period of time under hydrolytic conditions.

U.S. Patent No. 4,253,473 describes smoking tobacco compositions or substitute smoking tobacco compositions which upon smoking release substantially evenly and uniformly over an extended period of time.

U.S. Patent No. 3,818,107 describes a chewing gum containing a flavor release composition comprising polymer backbones with flavor groups appended therto. The flavor moieties are released from the polymer backbone by hydrolysis which is achievable by mastication of chewing gums containing the flavor groups.

As an alternative method, the flavoring chemicals may be covalently bound to an auxilliary component to form a higher molecular weight molecule of low volatility. The flavorant is released upon pyrolysis, heating or solvolysis of the tobacco or food product.

In general, inventions employing the second strategy use an ester or carbonate linkage of a higher molecular weight molecule to a alcoholic flavor molecule. In such a system, a flavor molecule is covalently bound to a polymer and may be depicted by the following generalized structure:

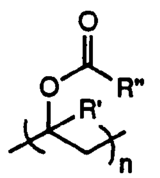

wherein R′ represents a lower alkyl group such as methyl, R" represents a flavorant radical such as menthyl and n is an integer of from 2 to 10,000.

This approach has been demonstrated in a number of U.S. Patents. For example, U.S. Patent No. 4,212,310 describes different flavored smoking tobacco products wherein some of the products contain an alcohol flavorant-release composition which delivers the flavor note of the alcohol upon pyrolysis.

U.S. Patent No. 4,119,106 describes alcohol flavorants-release polymeric derivatives which are designed to enhance tobacco smoke by releasing an alcohol flavorant to tobacco smoke without wasting the natural flavor of the resultant main stream tobacco smoke.

U.S. Patents Nos. 4,578,486 and 4,538,628 describe smoking tobacco compositions which contain dioxane diester flavorant-release additives. When subjected to normal smoking conditions such as cigarettes, the diester additive decomposes to release a volatile pyrolysis (alcohol or phenol) component which provides flavor-enhancing properties to the mainstream smoke and enhances the aroma of the sidestream smoke.

U.S. Patents Nos. 4,701,282, 4,538,627 and 4,540,004 describe the use of ketoester or carbonate compounds as flavorant additives which under cigarette smoking conditions pyrolyze to release flavorants which enhance the flavor of the mainstream smoke and the aroma of sidestream smoke.

Acetals have also been used as vehicles to covalently bind aldehyde flavorants. For example, U.S. Patent 4,296,137 describes the use of 1-ethoxy-1-ethanol acetate as a flavor or fragrance enhancer of a wide variety of consumable materials. The 1-ethoxy-1-ethanol acetate compound has the ability to liberate acetaldehyde in smoking tobacco.

U.S. Patent No. 4,280,011 describes the use of acetals as aldehyde generators in foodstuff applications.

U.S. Patent No. 3,625,709 describes food flavoring and aroma enhancers consisting of acetaldehyde combined with carbohydrates to form compositions which release acetaldehyde when combined with hot water or with cold water.

U.S. Patent No. 3,857,964 describes controlled release flavor compositions useful in flavor compositions which comprise flavor particles formed from a dispersion of flavor acetal or ketal in polymeric binders. The controlled release flavor compositions have multiple means of control, one of which is the hydrolysis of the flavor acetal or ketal. These controlled release flavor compositions are useful in chewing gums.

U.S. Patent Nos. 4,690,157 and 4,607,118 describe tobacco compositions which contain flavor release additives which, under cigarette smoking conditions, pyrolyze in a "retro-aldol" fragmentation reaction into products which enhance the flavor and aroma of the cigarette smoke.

The present invention provides organoleptic compositions which contain one or more compounds which, upon thermolysis, hydrolysis or both, release an odorant molecule. Exemplary of applications for the compositions of the present invention are tobacco products such as cigarettes and tobacco paper; foodstuffs such as cakes, cookies, crackers, breads and cereals; beverages such as tea and coffee; and gums such as chewing gums.

These compositions contain a dicarboalkoxy dioxolane derivative of formula

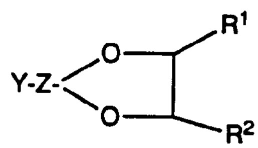

I

wherein

$R^1$ and $R^2$ are, independently, $-CO_2R^3$ wherein $R^3$ is -H or lower alkyl, provided that in at least one of $R^1$ and $R^2$, $R^3$ is lower alkyl; wherein Z is a direct bond, or $-CH=C(R^4)-$; where $R^4$ is -H or an alkyl group; and

Y is

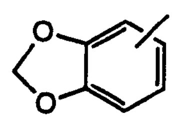

or

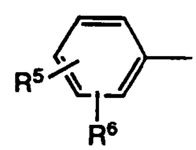

where $R^5$ and $R^6$ are independently -H, lower alkyl or $-OR^7$ where $R^7$ is -H or lower alkyl.

The compound or compounds are present in an amount such that, when subjected to hydrolysis, pyrolysis or both, provide an organoleptically effective level of flavor or aroma.

As used throughout this specification, the term "organoleptic" refers to compounds which stimulate the sense of smell or taste, and are thus perceived as having a characteristic odor, flavor or both.

The terms "odor", "fragrance" and "smell" are used interchangeably whenever a compound is referred to as an organoleptic which is intended to stimulate the sense of smell. The terms "flavor", "flavoring" and "flavorant" are also used interchangeably whenever an organoleptic compound is referred to which is intended to stimulate the sense of taste.

An "organoleptically effective amount" is a level or amount of a compound or compounds of the invention present in a material at which the incorporated compound or compounds exhibit a sensory effect.

The terms "tobacco" and "tobacco substitutes" are used in the conventional sense and include smokable as well as non-smokable forms in which tobacco is regularly used, e.g., cigarettes, snuff, chewable compositions and the like.

Alkyl (including the alkyl portion of alkoxy and alkylthio) - a branced or unbranched saturated carbon chain containing 1 to 12 carbon atoms with lower alkyl representing a chain containing 1 to 6 carbon atoms.

The organoleptic compositions of the invention contain one or more compounds which can be readily prepared by methods known to those skilled in the art. Exemplary methods are set forth in the example section below.

The usual method involves condensation of an aldehyde with diethyl tartrate (or some other lower alkyl tartrate) in an inert solvent in the presence of an acid catalyst. During the condensation water is usually removed.

Either protic or Lewis acids may be used. Some acids which may be used are p-toluenesulfonic acid, sulfuric acid, phosphoric acid, hydrochloric acid, methanesulfonic acid, pyridinium p-toluenesulfonate, ferric chloride, acidic clay, acidic ion exchange resins, zinc chloride and titanium tetrachloride.

Preferred acids include p-toluenesulfonic acid, methanesulfonic acid and pyridinium p-toluenesulfonate. The most preferred acid is p-toluenesulfonic acid.

A variety of inert solvents may be used, such as toluene, benzene, xylene, cyclohexane, hexane, dimethyl formamide, chlorobenzene and dichloroethane. The preferred solvents are toluene, xylene or dimethylformamide. The most preferred solvents are toluene and dimethylformamide.

The water formed in the reaction may be removed by azeotropic distillation or by interaction with a water scavenging agent such as a trialkyl orthoformate (alkyl is C1 to C5 and is usually the same as the lower alkyl in the tartrate), molecular sieves, sodium sulfate and the like.

In addition, the compounds may be prepared by first converting the aldehydes of the invention to the corresponding di-lower alkyl acetals (lower alkyl should be the same as the lower alkyl in the desired tartrate). Reaction of the acetals with a dialkyl tartrate under the conditions similar to that used when starting with an aldehyde will result in formation of the compounds.

Convenient methods of preparation are described in the following publications:

P. Kocienski et al., Synthetic Comm., 1984, 14, pp. 1087-1092

L.A. Paguette et al., J. Org. Chem., 1985, 50, pp. 5528-5533

M. Demuth et al., J. Am. Chem. Soc., 1986, 108, pp. 4149-4154

Y. Masaki et al., Chem. Letters, 1983, pp. 1835-1836

H. Yamamoto et al., J.Am. Chem. Soc., 1985, 107, pp. 8254-8256

T.W. Greene, "Protective Groups in Organic Synthesis", Chapter 4, John Wiley & Sons, New York, 1981

The organoleptic compositions may be used as flavorants in tobacco compositions, as sustained release odorants to mask or enhance the odors of burning tobacco products, in beverages, in microwaveable foods, and in the preparation of chewing gums.

The compounds used in the compositions are virtually odorless and tasteless under normal temperatures and atmospheric conditions, i.e., about 10-50 degrees Celcius and about 20 to 100% relative humidity, and exist as stable solids. However, when heated to higher temperatures, i.e., about 70 to about 300 degrees Celcius, in the presence of moisture or steam, they undergo a transformation in which the aldehyde is released.

Illustrative examples of preferred species and preferred isomers respectively are shown below:

(4R,5R)-2-(3-Ethoxy-4-hydroy-phenyl)-4,5-dicarboethoxy-1,3-dioxolane

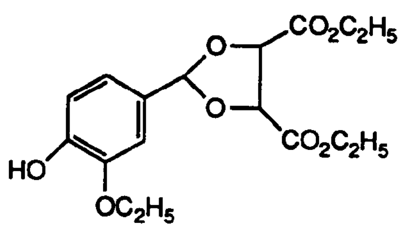

(4R,5R)-2-(3-Methoxy-4-hydroxy-phenyl)-4,5-dicarboethoxy-1,3-dioxolane

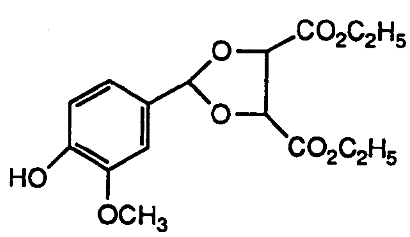

(4R,5R)-2-(4-Methoxyphenyl)-4,5-dicarboethoxy-1,3-dioxolane

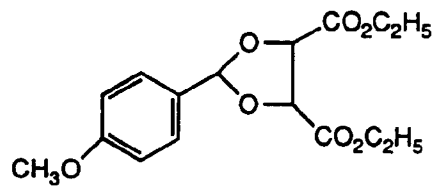

(4R,5R)-2-(4-Methylphenyl)-4,5-dicarboethoxy-1,3-dioxolane

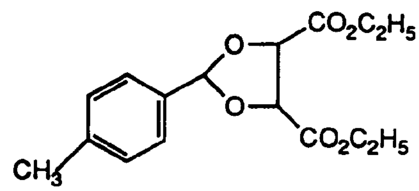

(4R,5R)-2-(2-Phenyl-1-ethenyl)-4,5-dicarboethoxy-1,3-dioxolane

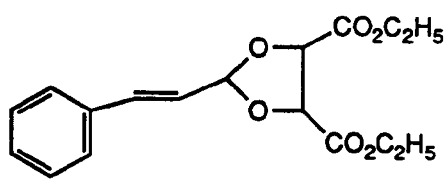

(4R,5R)-2-(3,4-Methylenedioxyphenyl)-4,5-dicarboethoxy-1,3-dioxolane

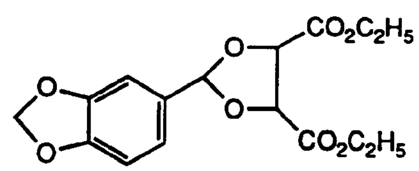

(4R,5R)-2-(3-Methoxy-4-hydroxy-phenyl)-4,5-dicarbomethoxy-1,3-dioxolane

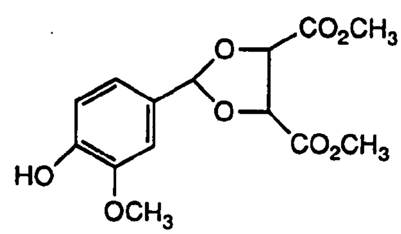

(E)-(4R,5R)-2-(1-Hexyl-2-phenyl-1-ethenyl)-4,5-dicarbomethoxy-1,3-dioxolane

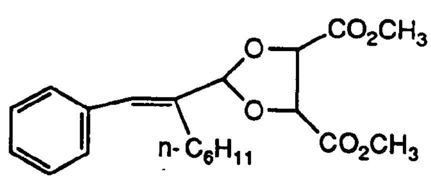

(E)-(4R,5R)-2-(1-Hexyl-2-phenyl-1-ethenyl)-4,5-dicarboethoxy-1,3-dioxolane

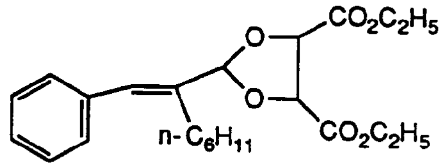

However, the compounds useful in the compositions of the present invention are not limited to any particular stereoisomer, and all possible stereoisomers are included within the scope of the invention.

The compounds of the present invention possess organoleptic properties and therefore permit the development of methods useful in enhancing the flavor of foods. These compounds are also useful in enhancing the odor, masking any unpleasant odor or enhancing the flavor of tobacco products.

Compositions of the present invention contain these compounds individually or in combination in an amount effective to enhance a characteristic flavor or odor of a material. More commonly, however, the compounds are mixed with other flavor or fragrance components in an amount sufficient to provide the desired flavor or odor characteristic.

The amount required to produce the desired, overall effect varies depending upon the particular compound chosen, the product in which it will be used, and the particular effect desired.

For example, depending upon the selection and concentration of the compound or compounds chosen, when added either singly or as a mixture to cigarette tobacco at levels ranging from about 5 ppm to about 50,000 ppm it tends to enhance the smoking flavor, mask undesirable smoking odor or both.

An important property of these compounds is that the flavorant or odorant is covalently bound as a nonvolatile compound and it is only when the tobacco product is ignited and burns that the flavorant or odorant is released.

These compounds are, conveniently, present either separately or as a mixture at levels ranging from about 5 ppm to about 50,000 ppm by weight on the media enclosing the tobacco which serves to incorporate the odorant or flavorant in the side-stream smoke as the tobacco product burns. Air borne flavorants, odorants or both along with other combustion products are thus introduced. This newly formed odorant or flavorant serves to enhance or mask the smoking odors depending upon selection and use levels of the compounds.

These compounds are particularly useful in the flavoring and aromatizing of certain cooked foods. For example, the compounds either singly or as a mixture added to cake batter impart an appropriate baking aroma to the cake as it is heated, as well as impart a flavor to the finished product. Typically, the compounds are employed at levels ranging from about 0.05 to about 5.00%.

The flavor of chewing gum may be enhanced by the addition of these compounds. A selected compound or mixture of compounds are kneaded into a gum base at levels ranging from about 0.1 to about 10.0% by weight. The appropriate flavors are released in the resulting gum upon mastication.

These compositions may, when used as additives to a foodstuff or tobacco product, contain or be added along with other ingredients. Such other ingredients include emulsifiers, carriers, binders, sweeteners, stabilizers, buffers and suitable solvents, etc.

The present invention relates also to particular novel dicarboalkoxy dioxolane derivatives as such.

The novel compounds are dicarboalkoxy dioxolane derivatives having the formula

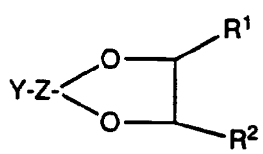 ,

wherein

$R^1$ and $R^2$ are, independently, $-CO_2R^3$ wherein $R^3$ is -H or lower alkyl, provided that in at least one of $R^1$ and $R^2$, $R^3$ is lower alkyl; wherein Z is a direct bond, or $-CH{=}C(R^4)-$; where $R^4$ is an alkyl group; and

Y is, when Z is a direct bond,

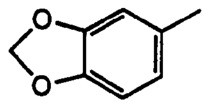

or

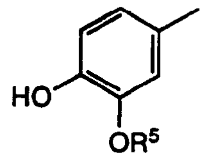

where $R^5$ is lower alkyl; and

Y, when Z is -CH=C($R^4$)-, is

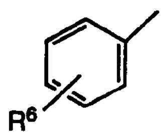

.

where $R^6$ is H, lower alkyl or $OR^7$, where $R^7$ is H or lower alkyl.

These compounds are prepared - as outlined above - by the condensation of an aldehyde

YZ-CHO (II)

or its di-lower alkyl acetal, with a lower alkyl, e.g. diethyl, tartrate

HO-$CHR^1$-$CHR^2$-OH (III)

, preferably in an inert solvent in the presence of an acid catalyst.

The following examples serve to illustrate embodiments of the invention and the advance over the prior art. The examples are presented to illustrate and not to limit the scope of the invention.

All parts, proportions, percentages and ratios used in the examples are by weight unless otherwise indicated.

Example 1

(4R-5R)-2-(3-Ethoxy-4-hydroxy-phenyl)-4,5-dicarboethoxy-1,3-dioxolane.

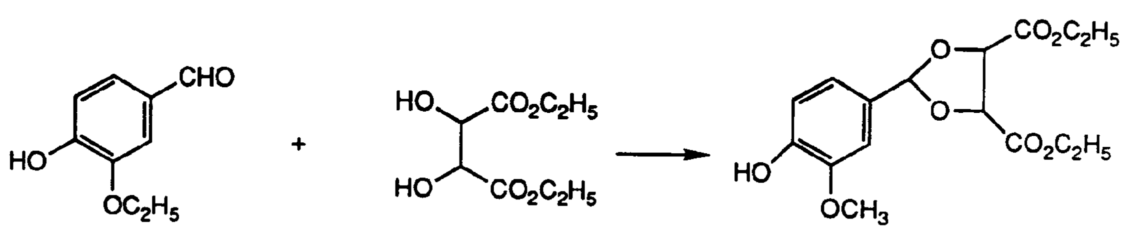

A mixture of diethyl L-tartrate (309 g, 1.5 mol), ethyl vanillin (166 g, 1 mol), toluene (2l) and p-toluenesulfonic acid (5 g, 0.026 mol) was heated at 115-116°C for 24 hours under a nitrogen atmosphere. During the reaction, water was removed by azeotropic distillation via a Dean-Stark trap. The mixture was washed sequentially with saturated sodium bicarbonate solution (500 ml), brine (2 x 1 l) and dried over sodium sulfate. The solvent was evaporated under reduced pressure and the solid obtained was recrystallized from isopropanol to provide 120 g (34% yield) of (4R,5R)-2-(3-ethoxy-4-hydroxy-phenyl)-4,5-dicarboethoxy-1,3-dioxolane, mp 78-80°C, $[\alpha]^{25}_{D}$ - 38.0° (c, 1.0, methanol). $^1$H-HMR ($CDCl_3$) $\delta$ 7.17 (1H, d,J=1.8 Hz), 7.06 (1H, dd,J=1.8 Hz and 8.10 Hz), 6.91 (1H, d,J=8.2 Hz), 6.07 (1H, s), 5.83 (1H, s), 4.91 (1H, d,J=4.0 Hz), 4.80 (1H, d,J=4.0 Hz), 4.37-4.25 (4H, 2q, overlapping, J=7.2 Hz), 4.15 (2H, q,J=7.0 Hz), 1.45 (3H, t,J=7.0 Hz), 1.38-1.29 (6H, 2t, overlapping J=7.2 Hz). IR (KBr) 3390, 2980. 2930, 1735, 1600 $cm^{-1}$. MS m/e (% abundance) 354 (55), 326 (3), 281(40), 182 (85), 167 (85), 154 (100), 137 (70), 110 (30), 93 (10), 81 (10), 53 (5).

Example 2

(4R,5R)-2-(3-Methoxy-4-hydroxy-phenyl)-4,5-dicarboethoxy-1,3-dioxolane.

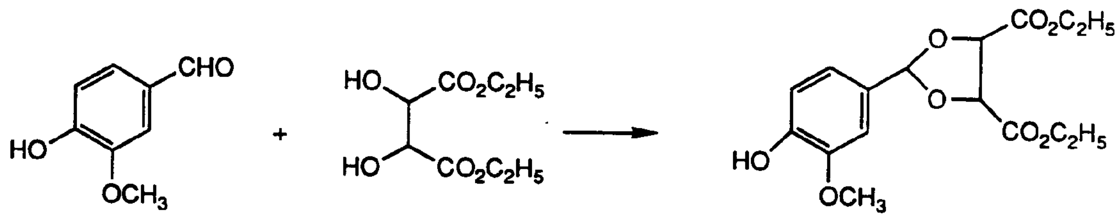

In a fashion similar to that described in Example 1, vanillin was condensed with diethyl L-tartrate to provide (4R,5R)-2-(3-methoxy-4-hydroxyphenyl)-4,5-dicarboethoxy-1,2-dioxolane,
mp 62-64°C, $[\alpha]_D^{25}$ -41.7° (c, 1.5, methanol). $^1$H-NMR ($CDCl_3$) δ 7.20 (1H, d,J=1.8 Hz), 7.03 (1H, dd,J=1.8 Hz and 8 Hz), 6.90 (1H, d,J=8 Hz), 6.09 (1H, s), 5.88 (1H, s), 4.92 (1H, d,J=3.8 Hz), 4.81 (1H, d,J=3.8 Hz), 4.36-4.24 (4H, 2q, overlapping, J=7.1 Hz), 3.90 (3H, s), 1.38-1.28 (6H, 2t, overlapping, J=7.1 HZ9. IR (KBr) 3500, 2970, 1740, 1605 $cm^{-1}$. MS m/e (% abundance) 340 (2), 267 (14), 168 (100),151 (95), 137 (50), 109 (10), 65 (10), 43 (6).

Example 3

(4R,5R)-2-(3,4-Methylenedioxyphenyl)-4,5-dicarboethoxy-1,3-dioxolane.

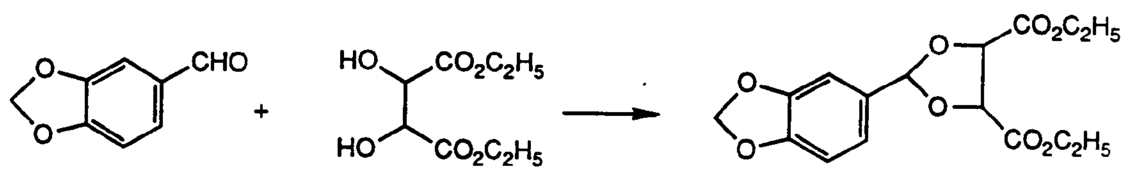

A mixture of piperonal (60 g, 0.4 mol), triethyl orthoformate (59.2 g, 0.4 mol), toluene (250 ml) and p-toluenesulfonic acid (2 g, 0.01 mol) was heated at 100-110°C for 0.5 h. Diethyl L-tartrate (103 g, 0.5 mol) was added to the hot solution over a 10 min. period. The mixture was then heated at reflux for 2 h. Subsequently over a 3 hour period, distillate (150 ml) was collected (pot temperature 84°C to 110°C). The mixture was cooled (25°C) and washed with sodium bicarbonate solution (2 x 50 ml) and brine (1 x 50 ml). Evaporation of solvents under reduced pressure provided 130.7 g of crude product.
Recrystallization from methanol provided 90 g (67% yield) of (4R,5R)-2-(3,4-methylenedioxyphenyl)-4,5-dicarboethoxy-1,3-dioxolane,
mp 39-40°C, $[\alpha]_D^{25}$ -34.8° (c, 1.0 methanol). $^1$H-NMR ($CDCl_3$) δ 7.12 (1H, d,J=1.5 Hz), 7.03 (1H, dd,J=1.5 Hz and 7.9 Hz), 6.80 (1H, d,J=7.9 Hz), 6.06 (1H, s), 5.97 (2H, s), 4.91 (1H, d,J=4.0 Hz), 4.80 (1H, d,J=4.0 Hz), 4.36-4.26 (4H, 2q, overlapping, J=7.0 Hz), 1.38-1.30 (6H, 2t, overlapping, J=7.0 Hz). IR (KBr) 2980, 2900, 1735, 1490, 1445, 1415 $cm^{-1}$. MS m/e (% abundance) 338 (3), 265 (16), 166 (100), 149 (96), 135 (64), 121 (35), 93 (10), 65 (12), 43 (8).

Example 4

(4R,5R)-2-(2-Phenyl-1-ethenyl)-4,5-dicarboethoxy-1,3-dioxolane.

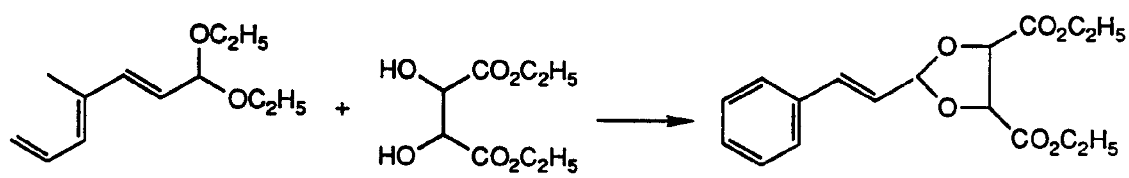

A mixture of cinnamaldehyde diethylacetal (223.0 g, 1.08 mol), diethyl L-tartrate (290 g, 1.41 mol), toluene (1100 g) and pyridinium p-toluenesulfonate (1.7 g) was heated at 92-110°C for a 6 hour period. During the heating period, volatiles (250-300 ml) were removed by distillation through an 8" Vigreux column. The mixture was cooled (25°C) and washed with aqueous sodium bicarbonate solution (350 ml) and brine (4 x 200 ml). Evaporation of solvents and crystallization of the residue gave 250 g (72.3% yield) of (4R,5R)-2-(2-phenyl-1-ethenyl)-4,5-dicarboethoxy-1,3-dioxolane,
mp 55.5-56°C, $[\alpha]_D^{25}$ -5.2° (c, 0.1, methanol). $^1$H-NMR($CDCl_3$) δ 7.45-7.26 (5H, m), 6.86 (1H, d,J=16.1 Hz), 6.26 (1H, dd,J=6.8 Hz and 16.1 Hz), 5.82 (1H, d,J=6.8 Hz), 4.87 (1H, d,J=3.6 Hz), 4.78 (1H, d,J=3.6 Hz), 4.36-4.26 (4H, 2q, overlapping J=7.1 Hz), 1.38-1.31 (6H, 2t, overlapping, J=7.1 Hz). IR (KBr) 2970, 2890, 1745, 1720, 1450, 1410 $cm^{-1}$. MS m/e (% abundance) 320 (2), 247 (6), 148 (38), 131 (78), 115 (100), 95 (80), 91 (8), 77 (15), 55 (14).

Example 5

(4R,5R)-2-(4-Methoxyphenyl)-4,5-dicarboethoxy-1,3-dioxolane.

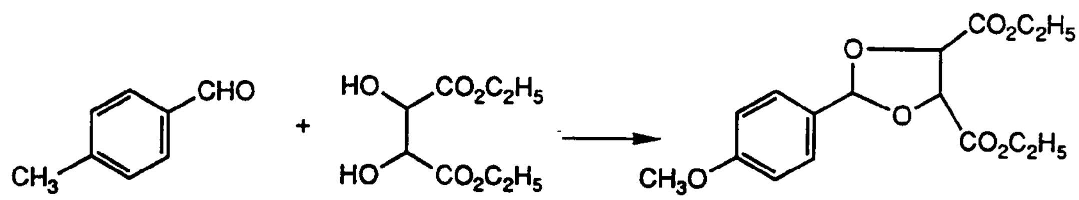

A mixture of triethylorthoformate (108 g, 0.74 mol) anisic aldehyde (89.4 g, 0.66 mol), diethyl L-tartrate (153.1 g, 0.743 mol), dimethylformamide (294 g) and p-toluenesulfonic acid (1.3 g) was heated at 100-105°C for 4 h. During the next 5 h, low boiling materials (100 ml) were removed by distillation (maximum pot temperature: 100°C, 300-360 mm vacuum). Sodium acetate (0.62 g) was added. Most of the dimethylformamide was then removed under reduced pressure (10 mm, maximum pot temperature 110°C). The residue was cooled (25°C) and diluted with toluene (500 ml). The mixture was washed with aqueous sodium bicarbonate (100 ml) and with brine (3 x 200 ml). Removal of solvents gave 211 g of crude product.
Chromatography of a portion of the crude product followed by Kugelrohr distillation (0.5 mm, bath temperature 240-250°C) gave a sample of (4R,5R)-2-(4-methoxyphenyl)-4,5-dicarboethoxy-1,3-dioxolane,
GLC purity: 98.6%, $[\alpha]_D^{25}$ -31.6° (c, 1.14, ethanol). $^1$H-NMR ($CDCl_3$) δ 7.52 (2H, d,J=7.5 Hz), 6.91 (2H, d,J=7.5 Hz), 6.11 (1H, s), 4.92 (1H, d,J=4.0 Hz), 4.81 (1H, dmJ=4.0 Hz), 4.37-4.25 (4H, 2q, overlapping, J=7.2 Hz), 3.81 (3H, s), 1.38-1.29 (6H, 2t, overlapping, J=7.2 Hz). IR (KBr) 2980, 1750, 1610, 1590, 1510 $cm^{-1}$. MS m/e (% abundance) 324 (2), 251 (12), 152 (92), 135 (100), 121 (40),108 (16), 91 (5), 77 (14), 51 (5).

Example 6

(4R,5R)-2-(4-Methylphenyl)-4,5-dicarboethoxy-1,3-dioxolane.

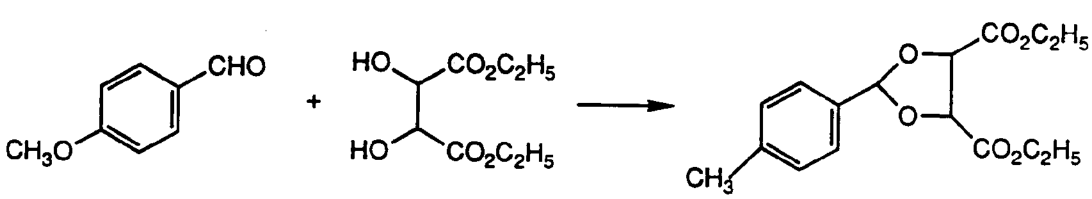

In a fashion similar to that described in Example 3, p-tolualdehyde was condensed with diethyl L-tartrate to provide (4R,5R)-2-(4-methylphenyl)-4,5-dicarboethoxy-1,3-dioxolane, an oil,
GLC analysis: 93%, $[\alpha]_D^{25}$ -9.6° (c, 0.1, methanol), $^1$H-NMR ($CDCl_3$) δ 7.47 (2H, d,J=8.0 Hz), 7.18 (2H, d,J=8.0 Hz), 6.12 (1H, s), 4.93 (1H, d,J=4.0 Hz), 4.81 (1H, d,J=7.2 Hz), 4.34-4.22 (4H, 2q, overlapping, J=7.2 Hz), 2.34 (3H, s), 1.35-1.26 (6H, 2t, overlapping, J=7.2 Hz). IR (film) 2980, 1740, 1615 $cm^{-1}$. MS m/e (% abundance) 308 (2), 293 (1), 279 (1), 235 (12), 136 (68), 119 (100), 105 (50), 91 (20), 77 (8), 43 (6).

Example 7

(4R,5R)-2-(3-Methoxy-4-hydroxy-phenyl)-4,5-dicarbomethoxy-1,3-dioxolane.

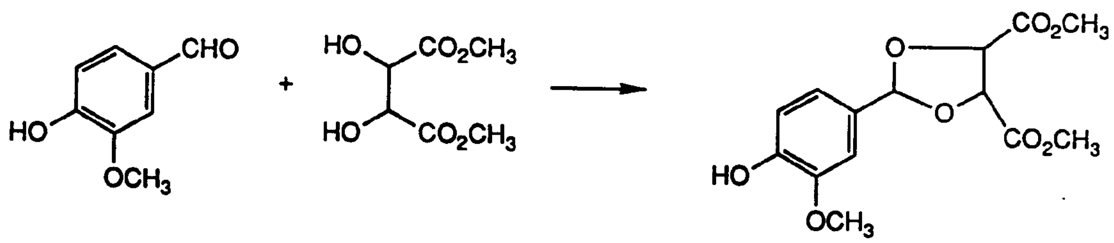

In a fashion similar to that described for Example 3, vanillin and dimethyl L-tartrate were condensed to provide (4R,5R)-2-(3-methoxy-4-hydroxy-phenyl)-4,5-dicarbomethoxy-1,3-dioxolane, mp 88-90°C, $[\alpha]_D^{25}$ -35.1° (c, 0.1, methanol). $^1$H-NMR ($CDCl_3$) δ 7.20 (1H, d,J=1.8 Hz), 7.06 (1H, dd,J=1.8 Hz and 8.2 Hz), 6.19 (1H, d,J=8.2 Hz), 6.08 (1H, s), 5.82 (1H, s), 4.96 (1H, d,J=3.8 Hz), 4.85 (1H, d,J=3.8 Hz), 3.91 (3H, s). 3.87 (3H, s), 3.84 (3H, s). IR (film) 3450, 1750, 1600, 1510, 1460, 1430 cm$^{-1}$. MS m/e (% abundance) 313 (2), 312 (12), 253 (40), 168 (84), 151 (100), 124 (22), 109 (14), 59 (18).

Example 8

(E)-(4R,5R)-2-(1-Hexyl-2-phenyl-1-ethenyl)-4,5-dicarbomethoxy-1,3-dioxolane.

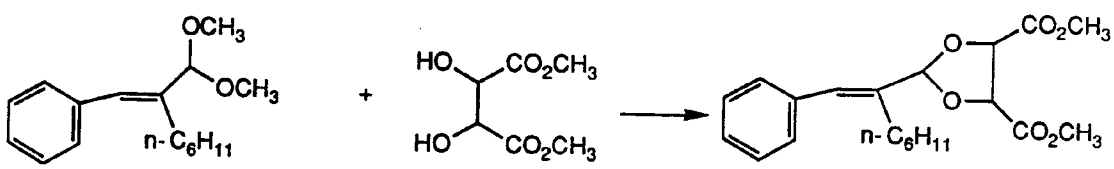

In a fashion similar to that described in Example 4, α-hexylcinnamaldehyde dimethyl acetal and dimethyl L-tartrate were condensed to provide a mixture of (E)- and (Z)-(4R,5R)-2-(1-hexyl-2-phenyl-1-ethenyl)-4,5-dicarbomethoxy-1,3-dioxolane in an approximate ratio of 90:10. Crystallization provided the pure E-isomer, mp 49-50°C, $[\alpha]_D^{25}$ -2.20 (c, 0.2, methanol). $^1$H-NMR ($CDCl_3$) δ 7.35-7.26 (5H, m), 6.74 (1H, s), 5.84 (1H, s), 4.86 (1H, d,J=4.4 Hz), 4.70 (1H, d,J=4.4 Hz), 3.85 (3H, s), 3.80 (3H, s), 2.34-2.27 (2H, m), 1.58-1.55 (2H, m), 1.37-1.26 (6H, m), 0.93-0.87 (3H, m). IR (KBr) 2900, 2840, 1730, 1430, 1340, 1200, 1100, 1060, 1030, 980, 950, 915, 870, 790, 750, 730, 6,90 cm$^{-1}$. MS m/e (% abundance) 305 (4), 292 (17), 291 (100), 145 (18), 142 (25), 131 (73), 129 (76), 128 (26), 117 (74), 116 (17), 115 (51), 104 (29), 91 (57), 59 (31), 41 (25).

Example 9

(E)-(4R,5R)-2-(1-Hexyl-2-phenyl-1-ethenyl)-4,5-dicarboethoxy-1,3-dioxolane.

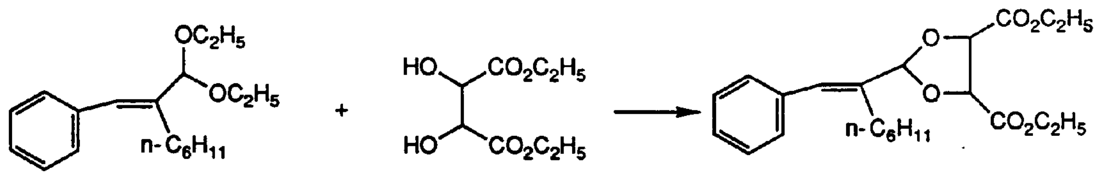

In a fashion similar to that described in Example 4, α-hexylcinnamaaldehyde diethyl acetal and diethyl L-tartrate were condensed to provide a mixture of (E)- and (Z)-(4R,5R)-2-(1-hexyl-2-phenyl-1-ethenyl)-4,5-dicarboethoxy-1,3-dioxolane. Low temperature crystallization provided the pure E-isomer, $[\alpha]_D^{25}$ -5.9 (c, 0.1, methanol). $^1$H-NMR ($CDCl_3$) δ 7.35-7.26 (5H, m), 6.75 (1H, s), 5.65 (1H, s), 4.87 (1H, d,J=4.5

Hz), 4.74 (1H, d,J=4.5 Hz), 4.36-4.25 (4H, 2q, overlapping, J=7.1 Hz), 2.38-2.32 (2H, m), 1.58-1.57 (2H, m), 1.38-1.25 (6H, 2t, overlapping, J=7.1 Hz), 1.38-1.22 (6H, m), 0.88-0.83 (3H, m). IR (film) 2900, 2840, 1750, 1450, 1360, 1260, 1200, 1100, 1010, 940, 900, 840, 730, 680 $cm^{-1}$. MS m/e (% abundance) 404 (0.6), 333 (4), 320 (18), 319 (100), 143 (21), 142 (38), 131 (80), 129 (88), 128 (28), 117 (78), 115 (52), 104 (31), 91 (56), 43 (27).

Example 10

Preparation of a Vanillin Cigarette

A 1% ethanolic solution of the compound from Example 1 was applied to cigarette papers at the rate of 100 ppm. The paper was incorporated into cigarettes. Prior to smoking, no odor of vanillin was observed. Upon smoking a strong, distinctly vanillin odor was observed in the room air.

Example 11

Preparation of a Cigarette Containing Vanillin Flavored Tobacco

A 1% ethanolic solution of the product of Example 1 was injected into the tobacco of a typical American Blend cigarette at a level of 100 ppm. Prior to smoking, no odor of vanillin was observed. Upon smoking, the mainstream and sidestream smoke displyed a strong vanillin odor.

Example 12

Preparation of an α-Hexyl Cinnamic Aldehyde Cigarette

A 1% ethanolic solution of the compound from Example 8 was applied to cigarette papers at the rate of 100 ppm. The paper was incorporated into cigarettes. Prior to smoking, no odor of α-hexyl cinnamic aldehyde was observed. Upon smoking a slight (but distinct), pleasant jasmine-like floral odor was observed in the room air.

Example 13

Preparation of Cinnamon Tea

The compound from Example 4 was added to tea bags containing unflavored green tea at the rate of 100 ppm. The tea bags had no cinnamon odor. On seeping, the headspace developed a distinct cinnamon aroma which provided a more pleasant tea.

## Claims

**Claims for the following Contracting States : CH, DE, FR, GB, IT, LI, NL**

1. Organoleptic compositions, comprising one or more dicarboalkoxy dioxolane derivatives of the formula

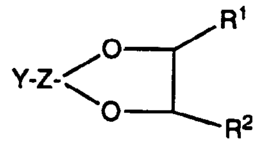

I

wherein

$R^1$ and $R^2$ are, independently, $-CO_2R^3$ wherein $R^3$ is -H or lower alkyl, provided that in at least one of $R^1$ and $R^2$, $R^3$ is lower alkyl; wherein Z is a direct bond, or $-CH=C(R^4)-$; where $R^4$ is -H or an alkyl group; and

Y is

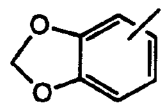 or 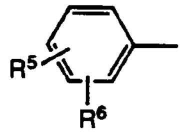

where $R^5$ and $R^6$ are independently -H, lower alkyl or $-OR^7$ where $R^7$ is -H or lower alkyl, which are present in an amount such that, when subjected to hydrolysis, pyrolysis or both, provide an organoleptically effective level of flavor or aroma.

2. The composition of claim 1 wherein the dicarboalkoxy compound is:
2-(3-ethoxy-4-hydroxy-phenyl)-4,5-dicarboethoxy-1,3-dioxolane,
2-(3-methoxy-4-hydroxy-phenyl)-4,5-dicarboethoxy-1,3-dioxolane,
2-(4-methoxyphenyl)-4,5-dicarboethoxy-1,3-dioxolane,
2-(4-methylphenyl)-4,5-dicarboethoxy-1,3-dioxolane,
2-(3,4-methylenedioxyphenyl)-4,5-dicarboethoxy-1,3-dioxolane,
2-(2-phenyl-1-ethenyl)-4,5-dicarboethoxy-1,3-dioxolane,
2-(3-methoxy-4-hydroxy-phenyl)-4,5-dicarbomethoxy-1,3-dioxolane,
2-(1-hexyl-2-phenyl-1-ethenyl)-4,5-dicarbomethoxy-1,3-dioxolane or
2-(1-hexyl-2-phenyl-1-ethenyl)-4,5-dicarboethoxy-1,3-dioxolane.

3. The composition of claim 2, wherein the dicarboalkoxy compound is:
(4R,5R)-2-(3-ethoxy-4-hydroxy-phenyl)-4,5-dicarboethoxy-1,3-dioxolane,
(4R,5R)-2-(3-methoxy-4-hydroxy-phenyl)-4,5-dicarboethoxy-1,3-dioxolane,
(4R,5R)-2-(4-methoxyphenyl)-4,5-dicarboethoxy-1,3-dioxolane,
(4R,5R)-2-(4-methylphenyl)-4,5-dicarboethoxy-1,3-dioxolane,
(4R,5R)-2-(3,4-methylenedioxyphenyl)-4,5-dicarboethoxy-1,3-dioxolane,
(4R,5R)-2-(2-phenyl-1-ethenyl)-4,5-dicarboethoxy-1,3-dioxolane,
(4R,5R)-2-(3-methoxy-4-hydroxy-phenyl)-4,5-dicarbomethoxy-1,3-dioxolane,
(E)-(4R,5R)-2-(1-hexyl-2-phenyl-1-ethenyl)-4,5-dicarbomethoxy-1,3-dioxolane or
(E)-(4R,5R)-2-(1-hexyl-2-phenyl-1-ethenyl)-4,5-dicarboethoxy-1,3-dioxolane.

4. A smoking composition which comprises natural tobacco or a tobacco substitute and an organoleptic composition of claims 1, 2 or 3.

5. The smoking composition of claim 4 wherein the concentration of the organoleptic composition is between about 5 ppm and about 50,000 ppm.

6. A media suitable for enclosing natural tobacco or a tobacco substitute, which contains between about 5 ppm and about 50,000 ppm of an organoleptic composition of claims 1, 2 or 3.

7. The media according to claim 6 wherein the organoleptic composition is present in an amount of from about 5 ppm to about 50,000 ppm.

8. A flavored food product comprising a foodstuff containing an organoleptic composition of claims 1, 2 or 3.

9. A flavored food product as described in claim 8, wherein the food product is baked.

10. The flavored food product according to claim 9 wherein the foodstuff is cake, cookie, cracker, bread or cereal.

11. A flavored gum comprising a gum base and an organoleptic composition of claims 1, 2 or 3.

12. A flavored beverage comprising an ingestible liquid and an organoleptic composition of claims 1, 2 or 3.

13. The use of the dicarboalkoxy dioxolane derivatives of formula I as given in any one of claims 1 to 3 as flavor or aroma precursors.

14. Dicarboalkoxy dioxolone derivatives of the formula:

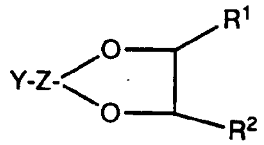

,

wherein

R¹ and R² are, independently, $-CO_2R^3$ wherein $R^3$ is -H or lower alkyl, provided that in at least one of $R^1$ and $R^2$, $R^3$ is lower alkyl; wherein Z is a direct bond, or $-CH=C(R^4)-$; where $R^4$ is an alkyl group; and

Y is, when Z is a direct bond,

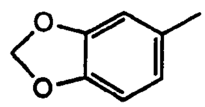

or

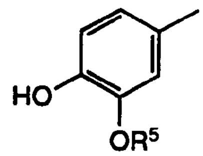

where $R^5$ is lower alkyl; and

Y, when Z is $-CH=C(R^4)-$, is

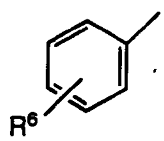

where $R^6$ is H, lower alkyl or $OR^7$, where $R^7$ is H or lower alkyl.

**15.** A compound selected from the group of
2-(3-ethoxy-4-hydroxy-phenyl)-4,5-dicarboethoxy-1,3-dioxolane,
2-(3-methoxy-4-hydroxy-phenyl)-4,5-dicarboethoxy-1,3-dioxolane,
2-(3,4-methylenedioxyphenyl)-4,5-dicarboethoxy-1,3-dioxolane,
2-(3-methoxy-4-hydroxy-phenyl)-4,5-dicarbomethoxy-1,3-dioxolane,
2-(1-hexyl-2-phenyl-1-ethenyl)-4,5-dicarbomethoxy-1,3-dioxolane and
2-(1-hexyl-2-phenyl-1-ethenyl)-4,5-dicarboethoxy-1,3-dioxolane.

**16.** A compound selected from the group of
(4R,5R)-2-(3-ethoxy-4-hydroxy-phenyl)-4,5-dicarboethoxy-1,3-dioxolane,
(4R,5R)-2-(3-methoxy-4-hydroxy-phenyl)4,5-dicarboethoxy-1,3-dioxolane,
(4R,5R)-2-(3,4-methylenedioxyphenyl)-4,5-dicarboethoxy-1,3-dioxolane,
(4R,5R)-2-(3-methoxy-4-hydroxy-phenyl)-4,5-dicarbomethoxy-1,3-dioxolane,
(E)-(4R,5R)-2-(1-heyl-2-phenyl-1-ethenyl)-4,5-dicarbomethoxy-1,3-dioxolane and
(E)-(4R,5R)-2-(1-hexyl-2-phenyl-1-ethenyl)-4,5-dicarboethoxy-1,3-dioxolane.

**17.** Process for the manufacture of the compounds of formula

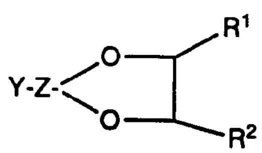

,

wherein

R¹ and R² are, independently, $-CO_2R^3$ wherein $R^3$ is -H or lower alkyl, provided that in at least one of $R^1$ and $R^2$, $R^3$ is lower alkyl; wherein Z is a direct bond, or $-CH=C(R^4)-$, where $R^4$ is an alkyl group; and

Y is, when Z is a direct bond,

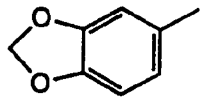

or

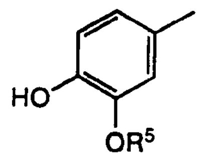

where $R^5$ is lower alkyl; and

Y, when Z is $-CH=C(R^4)-$, is

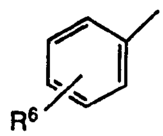

where $R^6$ is H, lower alkyl or $OR^7$, where $R^7$ is H or lower alkyl, comprising condensing an aldehyde

YZ-CHO (II)

or its di-lower alkyl acetal, with a lower alkyl tartrate

$HO-CHR^1-CHR^2-OH$ (III)

, preferably in an inert solvent in the presence of an acid catalyst.

**Claims for the following Contracting State : ES**

1. Organoleptic compositions, comprising one or more dicarboalkoxy dioxolane derivatives of the formula

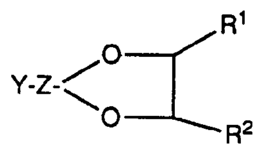

I

wherein

$R^1$ and $R^2$ are, independently, $-CO_2R^3$ wherein $R^3$ is -H or lower alkyl, provided that in at least one of $R^1$ and $R^2$, $R^3$ is lower alkyl; wherein Z is a direct bond, or $CH=C(R^4)-$; where $R^4$ is -H or an alkyl group; and

Y is

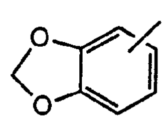

or

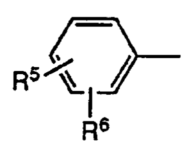

where $R^5$ and $R^6$ are independently -H, lower alkyl or $-OR^7$ where $R^7$ is -H or lower alkyl, which are present in an amount such that, when subjected to hydrolysis, pyrolysis or both, provide an organoleptically effective level of flavor or aroma.

2. The composition of claim 1 wherein the dicarboalkoxy compound is:
   2-(3-ethoxy-4-hydroxy-phenyl)-4,5-dicarboethoxy-1,3-dioxolane,
   2-(3-methoxy-4-hydroxy-phenyl)-4,5-dicarboethoxy-1,3-dioxolane,
   2-(4-methoxyphenyl)-4,5-dicarboethoxy-1,3-dioxolane,
   2-(4-methylphenyl)-4,5-dicarboethoxy-1,3-dioxolane,
   2-(3,4-methylenedioxyphenyl)-4,5-dicarboethoxy-1,3-dioxolane,
   2-(2-phenyl-1-ethenyl)-4,5-dicarboethoxy-1,3-dioxolane,

2-(3-methoxy-4-hydroxy-phenyl)-4,5-dicarbomethoxy-1,3-dioxolane,
2-(1-hexyl-2-phenyl-1-ethenyl)-4,5-dicarbomethoxy-1,3-dioxolane or
2-(1-hexyl-2-phenyl-1-ethenyl)-4,5-dicarboethoxy-1,3-dioxolane.

**3.** The composition of claim 2, wherein the dicarboalkoxy compound is:
(4R,5R)-2-(3-ethoxy-4-hydroxy-phenyl)-4,5-dicarboethoxy-1,3-dioxolane,
(4R,5R)-2-(3-methoxy-4-hydroxy-phenyl)-4,5-dicarboethoxy-1,3-dioxolane,
(4R,5R)-2-(4-methoxyphenyl)-4,5-dicarboethoxy-1,3-dioxolane,
(4R,5R)-2-(4-methylphenyl)-4,5-dicarboethoxy-1,3-dioxolane,
(4R,5R)-2-(3,4-methylenedioxyphenyl)-4,5-dicarboethoxy-1,3-dioxolane,
(4R,5R)-2-(2-phenyl-1-ethenyl)-4,5-dicarboethoxy-1,3-dioxolane,
(4R,5R)-2-(3-methoxy-4-hydroxy-phenyl)-4,5-dicarbomethoxy-1,3-dioxolane,
(E)-(4R,5R)-2-(1-hexyl-2-phenyl-1-ethenyl)-4,5-dicarbomethoxy-1,3-dioxolane or
(E)-(4R,5R)-2-(1-hexyl-2-phenyl-1-ethenyl)-4,5-dicarboethoxy-1,3-dioxolane.

**4.** A smoking composition which comprises natural tobacco or a tobacco substitute and an organoleptic composition of claims 1, 2 or 3.

**5.** The smoking composition of claim 4 wherein the concentration of the organoleptic composition is between about 5 ppm and about 50,000 ppm.

**6.** A media suitable for enclosing natural tobacco or a tobacco substitute, which contains between about 5 ppm and about 50,000 ppm of an organoleptic composition of claims 1, 2 or 3.

**7.** The media according to claim 6 wherein the organoleptic composition is present in an amount of from about 5 ppm to about 50,000 ppm.

**8.** A flavored food product comprising a foodstuff containing an organoleptic composition of claims 1, 2 or 3.

**9.** A flavored food product as described in claim 8, wherein the food product is baked.

**10.** The flavored food product according to claim 9 wherein the foodstuff is cake, cookie, cracker, bread or cereal.

**11.** A flavored gum comprising a gum base and an organoleptic composition of claims 1, 2 or 3.

**12.** A flavored beverage comprising an ingestible liquid and an organoleptic composition of claims 1, 2 or 3.

**13.** The use of the dicarboalkoxy dioxolane derivatives of formula I as given in any one of claims 1 to 3 as flavor or aroma precursors.

**14.** Process for the manufacture of the compounds of formula

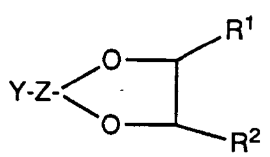

I

wherein

R¹ and R² — are, independently, $-CO_2R^3$ wherein $R^3$ is -H or lower alkyl, provided that in at least one of $R^1$ and $R^2$, $R^3$ is lower alkyl; wherein Z is a direct bond, or $-CH=C(R^4)-$; where $R^4$ is an alkyl group; and

Y — is, when Z is a direct bond,

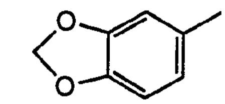 or 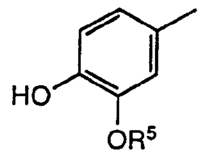

where $R^5$ is lower alkyl; and

Y, when Z is -CH=C($R^4$)-, is

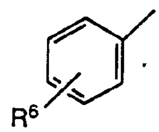

where $R^6$ is H, lower alkyl or $OR^7$, where $R^7$ is H or lower alkyl, comprising condensing an aldehyde

YZ-CHO (II)

or its di-lower alkyl acetal, with a lower alkyl tartrate

HO-$CHR^1$-$CHR^2$-OH (III)

, preferably in an inert solvent in the presence of an acid catalyst.

## Patentansprüche

### Patentansprüche für folgende Vertragsstaaten : CH, DE, FR, GB, IT, LI, NL

**1.** Organoleptische Kompositionen, enthaltend ein oder mehrere Dicarbalkoxydioxolan-derivate der Formel

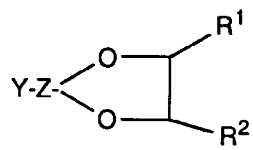

I

worin

$R^1$ und $R^2$ unabhängig voneinander -$CO_2R^3$ darstellen, worin $R^3$ -H oder nieder Alkyl sind, vorausgesetzt, dass mindestens einer der Reste $R^1$, $R^2$ und $R^3$ nieder Alkyl ist, und worin Z eine direkte Bindung oder die Gruppierung -CH=C($R^4$)- ist, worin $R^4$ -H oder ein Alkylrest ist, und

Y einer der Reste

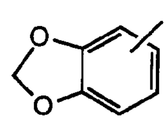 oder 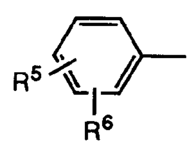

worin $R^5$ und $R^6$ unabhängig voneinander -H, nieder Alkyl oder -$OR^7$ sind, worin $R^7$ -H oder nieder Alkyl ist,

vorliegend in einer Menge, dass, wenn der Hydrolyse, Pyrolyse oder beiden Reaktionen unterworfen wird, eine für eine entsprechende Geschmackstoff- oder Riechstoffkomposition organoleptisch wirksame Menge entsteht.

**2.** Eine Komposition gemäss Anspruch 1, mit I ausgewählt aus der folgenden Gruppe:

2-(3-Ethoxy-4-hydroxy-phenyl)-4,5-dicarboethoxy-1,3-dioxolan,

2-(3-Methoxy-4-hydroxy-phenyl)-4,5-dicarboethoxy-1,3-dioxolan,

2-(4-Methoxyphenyl)-4,5-dicarboethoxy-1,3-dioxolan,
2-(4-Methylendioxyphenyl)-4,5-dicarboethoxy-1,3-dioxolan,
2-(3,4-Methylendioxyphenyl)-4,5-dicarboethoxy-1,3-dioxolan,
2-(2-Phenyl-1-ethenyl)-4,5-dicarboethoxy-1,3-dioxolan,
2-(3-Methoxy-4-hydroxy-phenyl)-4,5-dicarbomethoxy-1,3-dioxolan,
2-(1-Hexyl-2-phenyl-1-ethenyl)-4,5-dicarbomethoxy-1,3-dioxolan und
2-(1-Hexyl-2-phenyl-1-ethenyl)-4,5-dicarboethoxy-1,3-dioxolan.

3. Die Komposition gemäss Anspruch 2, mit I ausgewählt aus der folgenden Gruppe:
(4R,5R)-2-(3-Ethoxy-4-hydroxy-phenyl)-4,5-dicarboethoxy-1,3-dioxolan,
(4R,5R)-2-(3-Methoxy-4-hydroxy-phenyl)-4,5-dicarboethoxy-1,3-dioxolan,
(4R,5R)-2-(4-Methoxyphenyl)-4,5-dicarboethoxy-1,3-dioxolan,
(4R,5R)-2-(4-Methylphenyl)-4,5-dicarboethoxy-1,3-dioxolan,
(4R,5R)-2-(4-Methylendioxyphenyl)-4,5-dicarboethoxy-1,3-dioxolan,
(4R,5R)-2-(2-Phenyl-1-ethenyl)-4,5-dicarboethoxy-1,3-dioxolan,
(4R,5R)-2-(3-Methoxy-4-hydroxy-phenyl)-4,5-dicarbomethoxy-1,3-dioxolan,
(E)-(4R,5R)-2-(1-Hexyl-2-phenyl-1-ethenyl)-4,5-dicarbomethoxy-1,3-dioxolan und
(E)-(4R,5R)-2-(1-Hexyl-2-phenyl-1-ethenyl)-4,5-dicarboethoxy-1,3-dioxolan.

4. Ein Raucherartikel, enthaltend natürlichen Tabak oder ein Tabaksubstitut und eine organoleptisch wirksame Komposition einer der Ansprüche 1, 2 oder 3.

5. Raucherartikel gemäss Anspruch 4, worin die Konzentration der organoleptischen Komposition zwischen ungefähr 5 ppm und ungefähr 50'000 ppm beträgt.

6. Ein Medium zur Aufnahme von natürlichem Tabak oder eines Tabaksubstitutes, das zwischen ungefähr 5 ppm und ungefähr 50'000 ppm einer organoleptischen Komposition einer der Ansprüche 1, 2 oder 3 enthält.

7. Das Medium gemäss Anspruch 6, worin die organoleptische Komposition in einer Menge von ungefähr 5 ppm bis ungefähr 50'000 ppm beträgt.

8. Aromatisierte Lebensmittel mit einem Gehalt an einem Nahrungsmittel, enthaltend eine organoleptische Komposition einer der Ansprüche 1, 2 oder 3.

9. Aromatisierte Lebensmittel gemäss Anspruch 8, worin das Nahrungsmittel gebacken ist.

10. Aromatisierte Lebensmittel gemäss Anspruch 9, worin das Nahrungsmittel ein Kuchen, ein Cookie, ein Cracker, Brot oder eine Zerealie ist.

11. Ein aromatisierter Kaugummi, enthaltend eine Kaugummibase und eine organoleptische Komposition einer der Ansprüche 1, 2 oder 3.

12. Ein aromatisiertes Getränk, enthaltend eine trinkbare Flüssigkeit und eine organoleptische Komposition einer der Ansprüche 1, 2 oder 3.

13. Verwendung der Dicarboalkoxydioxolanderivate der Formel I gemäss Anspruch 1, 2 oder 3 als Riech- oder Geschmackstoffprekursoren

14. Dicarboalkoxydioxolanderivate der Formel

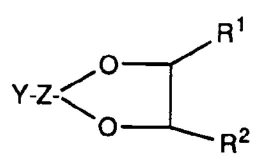

I

worin

$R^1$ und $R^2$ unabhängig voneinander $-CO_2R^3$ darstellen, worin $R^3$ -H oder nieder Alkyl sind, vorausgesetzt, dass mindestens einer der Reste $R^1$ und $R^2$ und $R^3$ nieder Alkyl ist, und worin Z

| | |
|---|---|
| | eine direkte Bindung oder die Gruppierung -CH=C($R^4$)- ist, worin $R^4$ -H oder ein Alkylrest ist, und |
| Y | , falls Z eine direkte Bindung ist, einer der Reste |

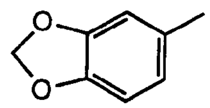

oder

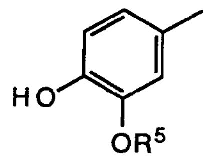

| | |
|---|---|
| | worin $R^5$ nieder Alkyl ist; und |
| Y | wenn Z -CH=C($R^4$)- ist, den Rest |

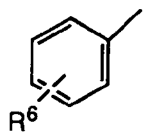

darstellt,
worin R6 -H, nieder Alkyl oder -$OR^7$ ist,
worin $R^7$ -H oder nieder Alkyl ist.

**15.** Eine Verbindung aus der Gruppe:

2-(3-Ethoxy-4-hydroxy-phenyl)-4,5-dicarboethoxy-1,3-dioxolan,
2-(3-Methoxy-4-hydroxy-phenyl)-4,5-dicarboethoxy-1,3-dioxolan,
2-(3,4-Methylendioxyphenyl)-4,5-dicarboethoxy-1,3-dioxolan,
2-(3-Methoxy-4-hydroxy-phenyl)-4,5-dicarbomethoxy-1,3-dioxolan,
2-(1-Hexyl-2-phenyl-1-ethenyl)-4,5-dicarbomethoxy-1,3-dioxolan und
2-(1-Hexyl-2-phenyl-1-ethenyl)-4,5-dicarboethoxy-1,3-dioxolan.

**16.** Eine Verbindung, ausgewählt aus der Gruppe:

(4R,5R)-2-(3-Ethoxy-4-hydroxy-phenyl)-4,5-dicarboethoxy-1,3-dioxolan,
(4R,5R)-2-(3-Methoxy-4-hydroxy-phenyl)-4,5-dicarboethoxy-1,3-dioxolan,
(4R,5R)-2-(3,4-Methylendioxyphenyl)-4,5-dicarboethoxy-1,3-dioxolan,
(4R,5R)-2-(3-Methoxy-4-hydroxy-phenyl)-4,5-dicarbomethoxy-1,3-dioxolan,
(E)-(4R,5R)-2-(1-Hexyl-2-phenyl-1-ethenyl)-4,5-dicarbomethoxy-1,3-dioxolan und
(E)-(4R,5R)-2-(1-Hexyl-2-phenyl-1-ethenyl)-4,5-dicarboethoxy-1,3-dioxolan.

**17.** Verfahren zur Herstellung der Verbindungen der Formel

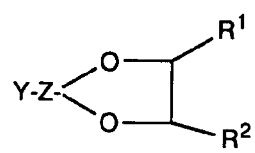

I

worin

| | |
|---|---|
| $R^1$ und $R^2$ | unabhängig voneinander -$CO_2R^3$ darstellen, worin $R^3$ -H oder nieder Alkyl sind, vorausgesetzt dass mindestens einer der Reste $R^1$ und $R^2$ und $R^3$ nieder Alkyl ist, und worin Z eine direkte Bindung oder die Gruppierung -CH=C($R^4$)- ist, worin $R^4$ -H oder ein Alkylrest ist und |
| Y | ,falls Z eine direkte Bindung ist, einer der Reste |

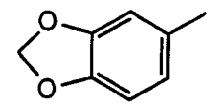 oder 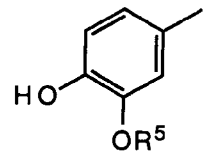

worin $R^5$ nieder Alkyl ist; und

Y wenn Z $-CH=C(R^4)-$ ist, den Rest

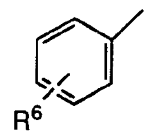

darstellt,
worin R6 -H nieder Alkyl oder $-OR^7$ ist,
worin $R^7$ -H oder nieder Alkyl ist,

dadurch gekennzeichnet, dass man einen Aldehyd der Formel

YZ-CHO (II)

oder eines seiner Di-nieder-alkyl-acetale mit einem Alkyltartrat der Formel

$HO-CHR^1-CHR^2-OH$ (III),

vorzugsweise in einem inertem Lösungsmittel in Anwesenheit eines sauren Katalysators, umsetzt.

**Patentansprüche für folgenden Vertragsstaat : ES**

1. Organoleptische Kompositionen, enthaltend ein oder mehrere Dicarbalkoxydioxolan-derivate der Formel

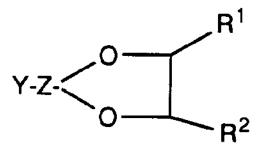 I

worin

$R^1$ und $R^2$ unabhängig voneinander $-CO_2R^3$ darstellen, worin $R^3$ -H oder nieder Alkyl sind, vorausgesetzt, dass mindestens einer der Reste $R^1$, $R^2$ und $R^3$ nieder Alkyl ist, und worin Z eine direkte Bindung oder die Gruppierung $-CH=C(R^4)-$ ist, worin $R^4$ -H oder ein Alkylrest ist, und

Y einer der Reste

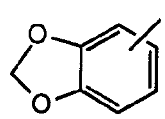 oder 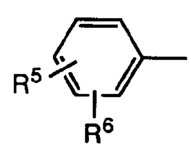

worin $R^5$ und $R^6$ unabhängig voneinander -H, nieder Alkyl oder $-OR^7$ sind, worin $R^7$ -H oder nieder Alkyl ist,

vorliegend in einer Menge, dass, wenn der Hydrolyse, Pyrolyse oder beiden Reaktionen unterworfen wird, eine für eine entsprechende Geschmackstoff- oder Riechstoffkomposition organoleptisch wirksame Menge entsteht.

2. Eine Komposition gemäss Anspruch 1, mit I ausgewählt aus der folgenden Gruppe:
   2-(3-Ethoxy-4-hydroxy-phenyl)-4,5-dicarboethoxy-1,3-dioxolan,
   2-(3-Methoxy-4-hydroxy-phenyl)-4,5-dicarboethoxy-1,3-dioxolan,
   2-(4-Methoxyphenyl)-4,5-dicarboethoxy-1,3-dioxolan,

2-(4-Methylendioxyphenyl)-4,5-dicarboethoxy-1,3-dioxolan,
2-(3,4-Methylendioxyphenyl)-4,5-dicarboethoxy-1,3-dioxolan,
2-(2-Phenyl-1-ethenyl)-4,5-dicarboethoxy-1,3-dioxolan,
2-(3-Methoxy-4-hydroxy-phenyl)-4,5-dicarbomethoxy-1,3-dioxolan,
2-(1-Hexyl-2-phenyl-1-ethenyl)-4,5-dicarbomethoxy-1,3-dioxolan und
2-(1-Hexyl-2-phenyl-1-ethenyl)-4,5-dicarboethoxy-1,3-dioxolan.

**3.** Die Komposition gemäss Anspruch 2, mit I ausgewählt aus der folgenden Gruppe:
(4R,5R)-2-(3-Ethoxy-4-hydroxy-phenyl)-4,5-dicarboethoxy-1,3-dioxolan,
(4R,5R)-2-(3-Methoxy-4-hydroxy-phenyl)-4,5-dicarboethoxy-1,3-dioxolan,
(4R,5R)-2-(4-Methoxyphenyl)-4,5-dicarboethoxy-1,3-dioxolan,
(4R,5R)-2-(4-Methylphenyl)-4,5-dicarboethoxy-1,3-dioxolan,
(4R,5R)-2-(4-Methylendioxyphenyl)-4,5-dicarboethoxy-1,3-dioxolan,
(4R,5R)-2-(2-Phenyl-1-ethenyl)-4,5-dicarboethoxy-1,3-dioxolan,
(4R,5R)-2-(3-Methoxy-4-hydroxy-phenyl)-4,5-dicarbomethoxy-1,3-dioxolan,
(E)-(4R,5R)-2-(1-Hexyl-2-phenyl-1-ethenyl)-4,5-dicarbomethoxy-1,3-dioxolan und
(E)-(4R,5R)-2-(1-Hexyl-2-phenyl-1-ethenyl)-4,5-dicarboethoxy-1,3-dioxolan.

**4.** Ein Raucherartikel, enthaltend natürlichen Tabak oder ein Tabaksubstitut und eine organoleptisch wirksame Komposition einer der Ansprüche 1, 2 oder 3.

**5.** Raucherartikel gemäss Anspruch 4, worin die Konzentration der organoleptischen Komposition zwischen ungefähr 5 ppm und ungefähr 50'000 ppm beträgt.

**6.** Ein Medium zur Aufnahme von natürlichem Tabak oder eines Tabaksubstitutes, das zwischen ungefähr 5 ppm und ungefähr 50'000 ppm einer organoleptischen Komposition einer der Ansprüche 1, 2 oder 3 enthält.

**7.** Das Medium gemäss Anspruch 6, worin die organoleptische Komposition in einer Menge von ungefähr 5 ppm bis ungefähr 50'000 ppm beträgt.

**8.** Aromatisierte Lebensmittel mit einem Gehalt an einem Nahrungsmittel, enthaltend eine organoleptische Komposition einer der Ansprüche 1, 2 oder 3.

**9.** Aromatisierte Lebensmittel gemäss Anspruch 8, worin das Nahrungsmittel gebacken ist.

**10.** Aromatisierte Lebensmittel gemäss Anspruch 9, worin das Nahrungsmittel ein Kuchen, ein Cookie, ein Cracker, Brot oder eine Zerealie ist.

**11.** Ein aromatisierter Kaugummi, enthaltend eine Kaugummibase und eine organoleptische Komposition einer der Ansprüche 1, 2 oder 3.

**12.** Ein aromatisiertes Getränk, enthaltend eine trinkbare Flüssigkeit und eine organoleptische Komposition einer der Ansprüche 1, 2 oder 3.

**13.** Verwendung der Dicarboalkoxydioxolanderivate der Formel I gemäss Anspruch 1, 2 oder 3 als Riech- oder Geschmackstoffprekursoren.

**14.** Verfahren zur Herstellung der Verbindungen der Formel

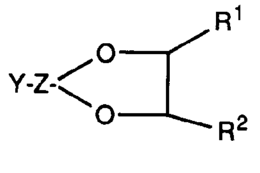

I

worin

$R^1$ und $R^2$ unabhängig voneinander $-CO_2R^3$ darstellen, worin $R^3$ -H oder nieder Alkyl sind, vorausgesetzt dass mindestens einer der Reste $R^1$ und $R^2$ und $R^3$ nieder Alkyl ist, und worin Z eine direkte Bindung oder die Gruppierung $-CH{=}C(R^4)-$ ist, worin $R^4$ -H oder ein Alkylrest

ist und

Y ,falls Z eine direkte Bindung ist, einer der Reste

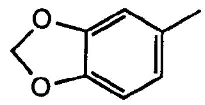

oder

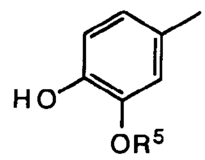

worin $R^5$ nieder Alkyl ist; und

Y wenn Z -CH=C($R^4$)- ist, den Rest

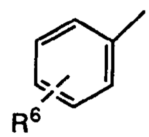

darstellt,
worin R6 -H nieder Alkyl oder -$OR^7$ ist,
worin $R^7$ -H oder nieder Alkyl ist,

dadurch gekennzeichnet, dass man einen Aldehyd der Formel

YZ-CHO (II)

oder eines seiner Di-nieder-alkyl-acetale mit einem Alkyltartrat der Formel

HO-$CHR^1$-$CHR^2$-OH (III),

vorzugsweise in einem inertem Lösungsmittel in Anwesenheit eines sauren Katalysators, umsetzt.

## Revendications

**Revendications pour les Etats contractants suivants : CH, DE, FR, GB, IT, LI, NL**

1. Compositions organoleptiques, qui comprennent un ou plusieurs dérivé(s) de dicarboxyalcoxydioxolane répondant à la formule :

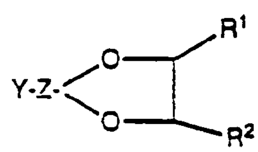

I

dans laquelle :
$R^1$ et $R^2$ représentent, indépendamment, le groupe -$CO_2R^3$ dans lequel $R^3$ est H ou un radical alkyle inférieur, à la condition qu'au moins l'un des radicaux $R^1$, $R^2$ et $R^3$ soient un alkyle inférieur ;
Z représente une liaison directe ou le groupe -CH=C($R^4$)- dans lequel $R^4$ représente H ou un radical alkyle ; et
Y représente :

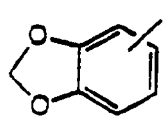

ou

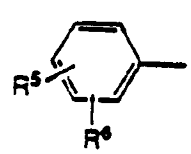

dans lequel $R^5$ et $R^6$ représentent indépendamment H, un radical alkyle inférieur ou -$OR^7$, $R^7$ étant H ou un radical alkyle inférieur,
qui sont présents en une quantité telle que, quand on soumet à l'hydrolyse, pyrolyse ou les deux, ces

compositions procurent un niveau organoleptiquement efficace de saveur ou d'arôme.

2. Composition selon la revendication 1, dans laquelle le composé dicarboalcoxy est choisi parmi les suivants :
2-(3-éthoxy-4-hydroxy-phényl)-4,5-dicarboéthoxy-1,3-dioxolane,
2-(3-méthoxy-4-hydroxy-phényl)-4,5-dicarboéthoxy-1,3-dioxolane,
2-(4-méthoxyphényl)-4,5-dicarboéthoxy-1,3-dioxolane,
2-(4-méthylphényl)-4,5-dicarboéthoxy-1,3-dioxolane,
2-(3,4-méthylènedioxyphényl)-4,5-dicarboéthoxy-1,3-dioxolane,
2-(2-phényl-1-éthényl)-4,5-dicarboéthoxy-1,3-dioxolane,
2-(3-méthoxy-4-hydroxy-phényl)-4,5-dicarbométhoxy-1,3-dioxolane,
2-(1-hexyl-2-phényl-1-éthényl)-4,5-dicarbométhoxy-1,3-dioxolane ou
2-(1-hexyl-2-phényl-1-éthényl)-4,5-dicarboéthoxy-1,3-dioxolane.

3. Composition selon la revendication 2, dans laquelle le composé dicarboalcoxy est l'un des suivants :
(4R,5R)-2-(3-éthoxy-4-hydroxy-phényl)-4,5-dicarboéthoxy-1,3-dioxolane,
(4R,5R)-2-(3-méthoxy-4-hydroxy-phényl)-4,5-dicarboéthoxy-1,3-dioxolane,
(4R,5R)-2-(4-méthoxyphényl)-4,5-dicarboéthoxy-1,3-dioxolane,
(4R,5R)-2-(4-méthylphényl)-4,5-dicarboéthoxy-1,3-dioxolane,
(4R,5R)-2-(3,4-méthylènedioxyphenyl)-4,5-dicarboéthoxy-1,3-dioxolane,
(4R,5R)-2-(2-phényl-1-éthényl)-4,5-dicarboéthoxy-1,3-dioxolane,
(4R,5R)-2-(3-méthoxy-4-hydroxy-phényl)-4,5-dicarbométhoxy-1,3-dioxolane,
(E)-(4R,5R)-2-(1-hexyl-2-phényl-1-éthényl)-4,5-dicarbométhoxy-1,3-dioxolane ou
(E)-(4R,5R)-2-(1-hexyl-2-phényl-1-éthényl)-4,5-dicarboéthoxy-1,3-dioxolane.

4. Composition à fumer qui comprend du tabac naturel ou un substitut de tabac et une composition organoleptique selon la revendication 1, 2 ou 3.

5. Composition à fumer selon la revendication 4, dans laquelle la concentration de la composition organoleptique est comprise entre environ 5 et 50.000 ppm.

6. Milieu qui convient pour entourer le tabac naturel ou un substitut de tabac, qui contient environ 5 à 50.000 ppm d'une composition organoleptique selon la revendication 1, 2 ou 3.

7. Milieu selon la revendication 6, dans lequel la composition organoleptique est présente à raison d'environ 5 à 50.000 ppm.

8. Produit alimentaire aromatisé qui comprend un produit alimentaire contenant une composition organoleptique selon la revendication 1, 2 ou 3.

9. Produit alimentaire aromatisé selon la revendication 8, dans lequel le produit alimentaire est cuit au four.

10. Produit alimentaire aromatisé selon la revendication 9, dans lequel le produit alimentaire est un gâteau, un cookie, un cracker, un pain ou une céréale.

11. Gomme aromatisée comprenant une base de gomme et une composition organoleptique selon la revendication 1, 2 ou 3.

12. Boisson aromatisée comprenant un liquide buvable et une composition organoleptique selon la revendication 1, 2 ou 3.

13. Utilisation de dérivés de dicarboalcoxy dioxolane de formule I selon l'une quelconque des revendications 1 à 3 en qualité de précurseurs de saveur ou d'arôme.

14. Dérivés de dicarboxyalcoxy-dioxolane de formule :

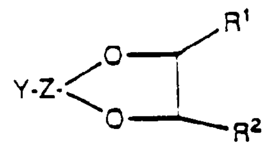

dans laquelle :

$R^1$ et $R^2$ représentent indépendamment le composé $-CO_2R^3$ dans lequel $R^3$ représente H ou un radical alkyle inférieur à la condition qu'au moins l'un des radicaux $R^1$, $R^2$ et $R^3$ soit un radical alkyle inférieur,
Z est une liaison directe ou le groupe $-CH=C(R^4)-$, dans lequel $R^4$ est un radical alkyle ; et
Y est, lorque Z est une liaison directe, le composé

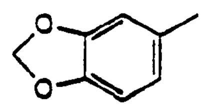

ou

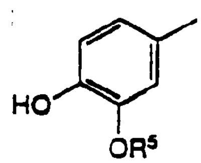

dans lequel $R^5$ est un radical alkyle inférieur ; et
quand Z est $-CH=C(R^4)-$, Y représente :

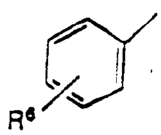

$R^6$ étant H, un radical alkyle inférieur ou $OR^7$ dans lequel $R^7$ est H ou alkyle inférieur.

**15.** Composé choisi dans le groupe comprenant les:
2-(3-éthoxy-4-hydroxy-phényl)-4,5-dicarboéthoxy-1,3-dioxolane,
2-(3-méthoxy-4-hydroxy-phényl-4,5-dicarboéthoxy-1,3-dioxolane,
2-(3,4-méthylènedioxyphenyl)-4,5-dicarboéthoxy-1,3-dioxolane,
2-(3-méthoxy-4-hydroxy-phényl)-4,5-dicarbométhoxy-1,3-dioxolane,
2-(1-hexyl-2-phényl-1-éthényl)-4,5-dicarbométhoxy-1,3-dioxolane
2-(1-hexyl-2-phényl-1-éthényl)-4,5-dicarboéthoxy-1,3-dioxolane.

**16.** Composé choisi parmi les suivants :
(4R,5R)-2-(3-éthoxy-hydroxy-phényl)-4,5-dicarboéthoxy-1,3-dioxolane,
(4R,5R)-2-(3-méthoxy-4-hydroxy-phényl)-4,5-dicarboéthoxy-1,3-dioxolane,
(4R,5R)-2-(3,4-méthylènedioxyphényl)-4,5-dicarboéthoxy-1,3-dioxolane,
(4R,5R)-2-(3-méthoxy-4-hydroxy-phényl)-4,5-dicarbométhoxy-1,3-dioxolane,
(E)-(4R,5R)-2-(1-hexyl-2-phényl-1-éthényl)-4,5-dicarbométhoxy-1,3-dioxolane
(E)-(4R,5R)-2-(1-hexyl-2-phényl-1-éthényl)-4,5-dicarboéthoxy-1,3-dioxolane.

**17.** Procédé de fabrication de composés de formule :

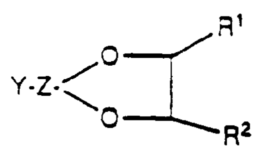

dans laquelle :

$R^1$ et $R^2$ représentent indépendamment le composé $-CO_2R^3$ dans lequel $R^3$ représente H ou un radical alkyle inférieur à la condition qu'au moins l'un des radicaux $R^1$, $R^2$ et $R^3$ soit un radical alkyle inférieur,
Z est une liaison directe ou le groupe $-CH=C(R^4)-$, dans lequel $R^4$ est un radical alkyle ; et

Y est, quand Z est une liaison directe,

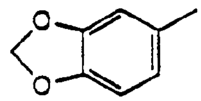

ou

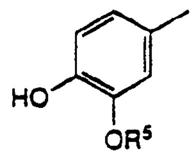

dans lequel $R^5$ est un radical alkyle inférieur et, quand Z représente $-CH=C(R^4)$, Y représente :

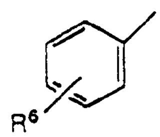

dans lequel $R^6$ est H, un radical alkyle inférieur ou $OR^7$ dans lequel $R^7$ est H ou un alkyle inférieur, qui consiste à condenser un aldéhyde

YZ-CHO (II)

ou son diacétal alkylique inférieur avec un tartrate d'alkyle inférieur :

$HO-CHR^1-CHR^2-OH$ (III)

de préférence dans un solvant inerte en présence d'un catalyseur acide.

**Revendications pour l'Etat contractant suivant : ES**

**1.** Compositions organoleptiques, qui comprennent un ou plusieurs dérivé(s) de dicarboxyalcoxydioxolane répondant à la formule :

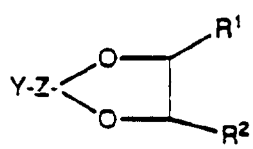

I

dans laquelle :
$R^1$ et $R^2$ représentent, indépendamment, le groupe $-CO_2R^3$ dans lequel $R^3$ est H ou un radical alkyle inférieur, à la condition qu'au moins l'un des radicaux $R^1$, $R^2$ et $R^3$ soient un alkyle inférieur ;
Z représente une liaison directe ou le groupe $-CH=C(R^4)-$ dans lequel $R^4$ représente H ou un radical alkyle ; et
Y représente :

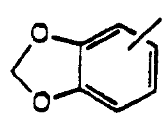

ou

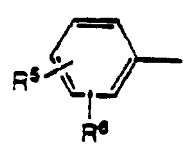

dans lequel $R^5$ et $R^6$ représentent indépendamment H, un radical alkyle inférieur ou $-OR^7$ $R^7$ étant H ou un radical alkyle inférieur,
qui sont présents en une quantité telle que, quand on soumet à l'hydrolyse, pyrolyse ou les deux, ces compositions procurent un niveau organoleptiquement efficace de saveur ou d'arôme.

**2.** Composition selon la revendication 1, dans laquelle le composé dicarboalcoxy est choisi parmi les suivants :

2-(3-éthoxy-4-hydroxy-phényl)-4,5-dicarboéthoxy-1,3-dioxolane,

2-(3-méthoxy-4-hydroxy-phényl)-4,5-dicarboéthoxy-1,3-dioxolane,
2-(4-méthoxyphényl)-4,5-dicarboéthoxy-1,3-dioxolane,
2-(4-méthylphényl)-4,5-dicarboéthoxy-1,3-dioxolane,
2-(3,4-méthylènedioxyphényl)-4,5-dicarboéthoxy-1,3-dioxolane,
2-(2-phényl-1-éthényl)4,5-dicarboéthoxy-1,3-dioxolane,
2-(3-méthoxy-4-hydroxy-phényl)-4,5-dicarbométhoxy-1,3-dioxolane,
2-(1-hexyl-2-phényl-1-éthényl)-4,5-dicarbométhoxy-1,3-dioxolane ou
2-(1-hexyl-2-phényl-1-éthényl)-4,5-dicarboéthoxy-1,3-dioxolane.

**3.** Composition selon la revendication 2, dans laquelle le composé dicarboalcoxy est l'un des suivants :
(4R,5R)-2-(3-éthoxy-4-hydroxy-phényl)-4,5-dicarboéthoxy-1,3-dioxolane,
(4R,5R)-2-(3-méthoxy-4-hydroxy-phényl)-4,5-dicarboéthoxy-1,3-dioxolane,
(4R,5R)-2-(4-méthoxyphényl)-4,5-dicarboéthoxy-1,3-dioxolane,
(4R,5R)-2-(4-méthylphényl)-4,5-dicarboéthoxy-1,3-dioxolane,
(4R,5R)-2-(3,4-méthylènedioxyphényl)-4,5-dicarboéthoxy-1,3-dioxolane,
(4R,5R)-2-(2-phényl-1-éthényl)-4,5-dicarboéthoxy-1,3-dioxolane,
(4R,5R)-2-(3-méthoxy-4-hydroxy-phényl)-4,5-dicarbométhoxy-1,3-dioxolane,
(E)-(4R,5R)-2-(1-hexyl-2-phényl-1-éthényl)-4,5-dicarbométhoxy-1,3-dioxolane ou
(E)-(4R,5R)-2-(1-hexyl-2-phényl-1-éthényl)-4,5-dicarboéthoxy-1,3-dioxolane.

**4.** Composition à fumer qui comprend du tabac naturel ou un substitut de tabac et une composition organoleptique selon la revendication 1, 2 ou 3.

**5.** Composition à fumer selon la revendication 4, dans laquelle la concentration de la composition organoleptique est comprise entre environ 5 et 50.000 ppm.

**6.** Milieu qui convient pour entourer le tabac naturel ou un substitut de tabac, qui contient environ 5 à 50.000 ppm d'une composition organoleptique selon la revendication 1, 2 ou 3.

**7.** Milieu selon la revendication 6, dans lequel la composition organoleptique est présente à raison d'environ 5 à 50.000 ppm.

**8.** Produit alimentaire aromatisé qui comprend un produit alimentaire contenant une composition organoleptique selon la revendication 1, 2 ou 3.

**9.** Produit alimentaire aromatisé selon la revendication 8, dans lequel le produit alimentaire est cuit au four.

**10.** Produit alimentaire aromatisé selon la revendication 9, dans lequel le produit alimentaire est un gâteau, un cookie, un cracker, un pain ou une céréale.

**11.** Gomme aromatisée comprenant une base de gomme et une composition organoleptique selon la revendication 1, 2 ou 3.

**12.** Boisson aromatisée comprenant un liquide buvable et une composition organoleptique selon la revendication 1, 2 ou 3.

**13.** Utilisation de dérivés de dicarboalcoxy dioxolane de formule I selon l'une quelconque des revendications 1 à 3 en qualité de précurseurs de saveur ou d'arôme.

**14.** Procédé de fabrication de composés de formule :

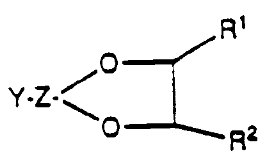

I

dans laquelle :
$R^1$ et $R^2$ représentent indépendamment le composé $-CO_2R^3$ dans lequel $R^3$ représente H ou un radical alkyle inférieur à la condition qu'au moins l'un des radicaux $R^1$, $R^2$ et $R^3$ soit un radical alkyle inférieur,

Z est une liaison directe ou le groupe -CH=C($R^4$)-, dans lequel $R^4$ est un radical alkyle ; et
Y est, lorque Z est une liaison directe, le composé

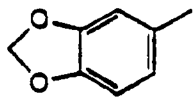

ou

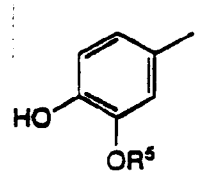

dans lequel $R^5$ est un radical alkyle inférieur ; et
quand Z est -CH=C($R^4$)-, Y représente :

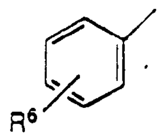

dans lequel $R^6$ représente H, un radical alkyle inférieur ou $OR^7$, $R^7$ étant H ou un alkyle inférieur, qui consiste à condenser un aldéhyde :

YZ-CHO (II)

ou son diacétal alkylique inférieur avec un tartrate d'alkyle inférieur :

HO-CHR$^1$-CHR$^2$-OH (III)

de préférence dans un solvant inerte en présence d'un catalyseur acide.